# EUROPEAN PATENT APPLICATION

(11) **EP 4 083 623 A1**
(43) Date of publication of application: **02.11.2022**
(21) Application number: 22170020.6
(22) Date of filing: 26.04.2022
(51) Int. Cl.: G01N 33/50, G01N 33/68

(54) **METHOD FOR THE IN VITRO DIAGNOSIS OF HEPATIC FIBROSIS AND KIT**

(30) Priority: 26.04.2021 IT 202100010454
(71) Applicant: Bocchietto, Elena, 28046 Meina (NO) (IT); Todeschi, Stefano, 28046 Meina (NO) (IT)
(72) Inventor: Bocchietto, Elena, 28046 Meina (NO) (IT); Todeschi, Stefano, 28046 Meina (NO) (IT)
(74) Representative: Trupiano, Federica

(57) **Abstract**

The present invention relates to a method to perform an in vitro diagnosis of hepatic fibrosis, performed on samples isolated from the subject to be subjected to said diagnosis. Furthermore, the present invention relates to a kit that allows to perform such a method of *in vitro* diagnosis and the use of biomarker panels in the method and kit of the invention.

## Description

### ABSTRACT

The present invention relates to a method to perform an *in vitro* diagnosis of hepatic fibrosis, performed on samples isolated from the subject to be subjected to said diagnosis. Furthermore, the present invention relates to a kit that allows to perform such a method of *in vitro* diagnosis and the use of biomarkers in the method and kit of the invention.

### BACKGROUND ART

The cholestatic liver disorders represent one of the major causes of fibrosis and cirrhosis in the patients with acute and chronic liver damage. The cholestasis is a condition in which bile cannot flow from the liver to the duodenum and there is stasis with retrograde flow of the bile to the blood, either because of inability to secrete to the biliary bed or because of increased downstream pressure. The cholestasis can cause fibrosis of the liver and result in primary biliary cirrhosis or sclerosing cholangitis. These disorders can be caused by genetic defects, mechanical aberrations, toxins, or malfunctions in the immune system that damage the bile ducts, causing a buildup of bile and tissue damages to the liver. In the early stages, it is asymptomatic and difficult to detect.

In general, in the hepatic fibrosis there is an excessive accumulation of connective tissue in the liver; this tissue represents the scarring result in response to the chronic and repeated liver cell damage. Generally, the fibrosis progresses, thus disrupting the liver architecture and eventually the liver function, while the hepatocyte regeneration attempts to replace and repair the damaged tissue. Various types of chronic liver damage can cause fibrosis, including non-alcoholic steatosis, viral hepatitis, excessive alcohol consumption and an unbalanced diet.

At present, the diagnosis of hepatic fibrosis is carried out by liver biopsy. The liver biopsy is an invasive practice that is often performed when the fibrosis affecting the liver is already in an advanced stage and, therefore, does not allow an early diagnosis of the disease. Furthermore, the liver biopsy has several limitations as well as complications, and does not always efficiently reflect the fibrotic changes occurring in the liver, as an optimally sized biopsy contains 5-11 complete portal spaces but reflects only 1/50000 of the liver volume. Furthermore, the rapid development of new therapies for the treatment of certain liver diseases increases the demand for additional and frequent hepatic fibrosis evaluations. The measurement of the liver hardness by Fibroscan, for example, is a non-invasive, rapid, and low-cost elastographic method for assessing the level of hepatic fibrosis. However, Fibroscan can only show the absence of fibrosis or estimate the degree of fibrosis and cannot be performed on all patients and is a useful but late clinical evidence, as it cannot detect the etiological phenomenon at birth.

Therefore, there is a need to fine-tune a method that enables early diagnosis of cholestatic liver disorders and that is both non-invasive and reliable from a biological but also from a clinical point of view.

At present, the diagnosis by the use of serum or urinary biomarkers is based on indirect biomarkers and direct biomarkers. The indirect biomarkers reflect alterations in the liver function, which are induced by the fibrosis, while the direct biomarkers reflect the changes induced on the extracellular matrix by the fibrogenesis. Examples of indirect biomarkers are: GOT/GPT ratio (AST/ALT RATIO), platelet count, prothrombin time, APRI (AST/Platelet Ratio Index), FIB-4. To date, the identification of said indirect biomarkers of hepatic fibrosis does not allow to make a sure and early diagnosis of fibrosis because their modification is related to severe organ damages which by then are many times irreparable.

The direct biomarkers of fibrosis comprise a variety of serum or urinary markers that are directly involved in the deposition or removal of extracellular matrix (ECM). Examples of direct biomarkers are: hyaluronic acid, PIIINP, laminin, metal-proteinase (MMP) and metal-proteinase inhibitors (TIMPs), TGF-β, and TNF-α.

The limitations of clinical use of the aforementioned direct biomarkers are that the technique to detect them is complex and not available in all testing centers. Furthermore, they are not liver-specific biomarkers, potentially being able to correlate with fibrogenic processes occurring in other organs, and their serum or urinary concentration is strictly dependent on the renal excretion and hepatic metabolism.

In light of this, there is an increasing need to fine-tune a diagnosis method of hepatic fibrosis, in particular cholestasis-induced hepatic fibrosis, that is non-invasive, specific and sufficiently sensitive to determine with certainty and well in advance the etiology so to allow the clinician to intervene in time with diets and therapies.

### OBJECTS OF THE INVENTION

Object of the present invention is to provide a method which enables in a short time to make an *in vitro* diagnosis of hepatic fibrosis, in particular, but not only, cholestasis-induced hepatic fibrosis, in a subject.

Another object of the present invention is to provide a method which enables to make an *in vitro* diagnosis of hepatic fibrosis at an early stage.

A further object of the present invention is to provide a kit which enables to make an *in vitro* diagnosis of hepatic fibrosis, in particular, but not only, cholestasis-induced hepatic fibrosis, in a subject at an early stage.

### DESCRIPTION OF THE INVENTION

The objects above, as well as other objects, are achieved by the present invention, subject-matter of which is a method comprising the determination of the protein level of a group of proteins identified as over-regulated or down-regulated in case of hepatic fibrosis, in particular in case of cholestasis-induced hepatic fibrosis. The method of the invention is particularly useful for the diagnosis of hepatic fibrosis at an early stage. The group of proteins useful for the method of the invention has been identified by the Applicant through an original interpretive method based on an algorithm that has been constructed on the basis of experimental studies in an animal model reproducing more or less early stages of hepatic fibrosis. The serum from the animals in which hepatic fibrosis was induced was analyzed for a wide range of soluble proteins (96) by means of a generic commercially available non-diagnostic kit, and by comparison with the untreated animals it was possible to identify the proteins involved in the inflammatory and fibrotic process, that show a statistically significant increase or decrease in expression at short (8 days) or medium (15 days) times after the disease was induced. The statistical and combined analysis of these data by means of an algorithm (multivariate and univariate analysis, t-test and volcano plot) has allowed to identify 5 molecules strongly involved in the early development of the disease, and other 11 molecules modulated in a less marked but still significant way both at early and more advanced level of the development of the experimental model of fibrosis.

Thus, according to one of its aspects, subject-matter of the invention is a method for making an *in vitro* diagnosis of hepatic fibrosis, comprising:
a. measuring the levels of at least 3 proteins, preferably at least 5 proteins, selected from
   Osteoprotegerin, JAM-A, RANTES, sTNF RI, IL20, GITR, CD36/SRB3, CD30, Granzyme B, Gas 6, MFG-E8, CXCL16, Pro-MMP9, MIP-3a and TWEAK R, in one or more isolated biological samples of a subject;
b. comparing said levels with one or more reference values, said reference values being the levels of said proteins in a healthy subject and/or in a subject suffering from hepatic fibrosis;
   wherein
   - the over-regulation of said at least 3 proteins, preferably at least 5 proteins, with respect to the levels of said proteins in a healthy subject, and/or
   - the substantial equivalence of the levels of said at least 3 proteins, preferably at least 5 proteins, with respect to the levels of said proteins in a subject suffering from hepatic fibrosis,
   are indicative of hepatic fibrosis and/or predisposition or progression of the condition of hepatic fibrosis.

According to an embodiment, in step (a) the method also comprises measuring KC protein levels, wherein
- the down-regulation of said protein with respect to the levels of said protein in a healthy subject, and/or
- the substantial equivalence of the levels of said protein with respect to the levels of said protein in a subject suffering from hepatic fibrosis,
are indicative of hepatic fibrosis and/or predisposition or progression of the condition of hepatic fibrosis.

According to the present invention, by "early diagnosis" is meant the ability to detect a predisposition to the onset of the disease before clinical evidence of liver disease is recognized, such as, for example, histological evidence or a marked increase in alkaline phosphatase, bilirubin and other liver enzyme markers in the serum. According to the present invention, by "measuring the levels" is meant to quantitatively determine the levels of said proteins in said biological samples. According to the present invention, by "biological samples" is preferably meant isolated blood samples, i.e., peripheral blood or serum or plasma, but other types of biological samples may be used, for example saliva or urine.

The proteins used in the method of the invention are described in the literature.

The abbreviations or acronyms used herein to denote the proteins are well known to the skilled in the art. Details on the proteins corresponding to said abbreviations or acronyms may, for example, be found in databases available on-line, for example at https://www.ncbi.nlm.nih.gov/protein/.

Specifically JAM-A= Junctional Adhesion Molecule A; RANTES= Regulated upon Activation, Normal T Cell Expressed and Presumably Secreted; sTNF RI = soluble Tumor Necrosis Factor Receptor I; IL 20= Interleukin 20; GITR = glucocorticoid-induced TNFR-related protein; CD36/SRB3= scavenger receptor class B, member; CD30=TNF Receptor Superfamily Member 8; Gas 6= growth Arrest-Specific protein 6; MFG-E8 = Milk fat globule-EGF factor 8 protein, also known as Lactadherin; CXCL16= C-X-C Motif Chemokine Ligand 16; Pro-MMP9= latent form of Matrix metallopeptidase 9; MIP-3a =Macrophage Inflammatory Protein-3 alpha; TWEAK R= TNF-related Weak Inducer of Apoptosis Receptor; KC=keratinocyte-derived chemokine; TARC = thymus- and activation-regulated chemokine

The term "over-regulation" herein denotes a higher level of the said proteins than the reference values in the healthy subject. The term "under-regulation" denotes a lower level.

The term "substantial equivalence" herein denotes that a level of the said proteins is not significantly different from the reference values in the subject suffering from hepatic fibrosis.

According to a preferred embodiment, said reference values are determined in samples of one or more healthy subjects or one or more subjects suffering from liver fibrosis or, alternatively, said reference values may be derived from manuals, publicly available databases or the scientific literature.

According to an embodiment, the comparison of step (b) can be performed either manually or by processing through programs comprising predictive models. According to a preferred embodiment, the method of the invention comprises:
a'. measuring the levels of at least 3 proteins, preferably at least 5 proteins, selected from
   Osteoprotegerin, JAM-A, RANTES, sTNF RI, IL20, GITR, CD36/SRB3, CD30, Granzyme B, Gas 6, MFG-E8, CXCL16, Pro-MMP9, MIP-3 and TWEAK R, in one or more isolated biological samples of a subject;
b'. comparing the said levels with one or more reference values, as defined above, resulting in comparison data; and/or
c'. processing the comparison data obtained from step (b') by using spreadsheets or an analysis program comprising a predictive mathematical model;
   wherein
      - the over-regulation of said at least 3 proteins, preferably at least 5 proteins, with respect to the levels of said proteins in a healthy subject, and/or
      - the substantial equivalence of said at least 3 proteins, preferably at least 5 proteins, with respect to the levels of said proteins in a subject suffering from hepatic fibrosis,
   are indicative of hepatic fibrosis and/or progression of the disease of hepatic fibrosis. According to an embodiment, in step (a) or (a') the method also provides for measuring KC protein levels, wherein
      - the down-regulation of said protein with respect to the levels of said protein in a healthy subject, and/or
      - the substantial equivalence of the levels of said protein with respect to the levels of said protein in a subject suffering from hepatic fibrosis,
   are indicative of hepatic fibrosis and/or predisposition or progression of the condition of hepatic fibrosis.

According to a preferred embodiment, in step (a) or (a') the levels of at least 5 proteins selected from Osteoprotegerin, JAM-A, RANTES, sTNF RI, IL20 GITR, CD36/SRB3, CD30, Granzyme B and Gas 6 are measured.

More preferably, the levels of the proteins Osteoprotegerin, JAM-A, RANTES, sTNF RI and CD36/SRB3, the latter being strongly over-regulated in the early phase of the disease, are measured.

According to an embodiment, in step (a) or (a') the levels of at least 5 proteins selected from Osteoprotegerin, JAM-A, RANTES, sTNF RI, IL20 GITR, CD36/SRB3, CD30, Granzyme B, Gas 6 and also the KC protein are measured.

More preferably, the levels of the proteins Osteoprotegerin, JAM-A, RANTES, sTNF RI and CD36/SRB3 and also the KC protein are measured.

According to an embodiment, in step (b) or (b') said levels are compared with one or more reference values, said reference values being the levels of said proteins in a healthy subject.

According to an embodiment, in step (b) or (b') said levels are compared with one or more reference values, said reference values being the levels of said proteins in a subject suffering from hepatic fibrosis.

In particular, the method of the invention allows to measure, in one or more samples isolated from the subject to be subjected to investigation, the level of the aforementioned proteins which have been shown to be specific biomarkers of hepatic fibrosis based on studies performed on model mice of hepatic fibrosis and on healthy mice (Experimental Section below, Example 2).

The method of the invention may advantageously be performed on biological samples, preferably blood samples, as defined above, isolated from the subject to be subjected to investigation for the presence of hepatic fibrosis, in particular cholestasis-induced fibrosis. Alternatively, said biological samples may be saliva or urine.

The method according to the invention may comprise determining the proteins stated in step (a) or (a') of the method.

The measurement of phase (a) or (a') can be made by any appropriate method. For example, methods using protein microarrays, immunoassays such as ELISA, EIA, RIA, western blot, protein array, immunochemistry and immunohistochemistry methods are known in the art.

According to a preferred embodiment, step (a) or (a') of the method is carried out by ELISA assay (*enzyme-linked immunosorbent assay*). In this case, the level of protein expression is detected by ELISA technique. According to the invention, it is possible to use each of the variants of the ELISA assay to carry out step (a) or (a') of the method, i.e., direct ELISA, indirect ELISA, sandwich ELISA and competitive ELISA, according to known practices.

According to another preferred embodiment, step (a) or (a') of the method is carried out on an analysis platform of the protein level, preferably a platform of protein array. Once the protein level is obtained, said level can be advantageously compared with the reference values for said proteins, for example detected in reference samples so to obtain comparison data (step (b) or (b') of the method of the invention).

According to the present invention, the "comparison data" are the data obtained by comparing the quantitative level of the proteins detected in said samples isolated from the subject to be subjected to investigation and the reference values for said proteins. In particular, the comparison data according to step (b) or (b') of the method are obtained by calculating the ratio between the protein levels detected in the sample isolated from the subject to be subjected to investigation and the reference values for said proteins.

In a further embodiment of the method, the protein level of one or more of said proteins in one or more reference samples according to step (b) or (b') of the method may be obtained by consulting an artificial intelligence (AI)-based or machine learning (ML)-based algorithm that serves as an aid to the early diagnosis.

The comparison of the protein level of the aforementioned proteins to the reference level will allow to establish a quantitative difference or substantial equivalence of said proteins, depending on the type of comparison made, which will be useful in making a diagnosis.

In an embodiment, the reference value according to step (b) or (b') of the method is determined from a sample other than that to be subjected to investigation. In the present patent application said sample, in which the reference value is determined, is referred to as the "reference sample".

When the reference sample is a sample isolated from a healthy subject, i.e. one not suffering from any liver disease, the method of the invention will allow to establish a diagnosis of hepatic fibrosis in case the level of one or more of the proteins according to step (a) or (a') of the method, in the samples isolated from the subject to be subjected to investigation, is found to be increased (over-regulated for all proteins with the exception of the under-regulated KC protein) with respect to the level of the same proteins in the reference samples. In fact, the reference sample in this case has a physiological level of proteins, i.e. is not altered.

In contrast, when the reference sample is a sample isolated from a subject suffering from liver diseases affected by fibrosis, the method of the invention will allow to establish a diagnosis of hepatic fibrosis in case the level of the proteins according to step (a) or (a') of the method, in the samples isolated from the subject to be subjected to investigation, is the same or similar with respect to the level of the same proteins in the reference samples.

As mentioned, the data concerning the quantitative difference of the proteins detected in samples subjected to investigation and reference samples can be advantageously processed in a program equipped with a predictive model capable of integrating the data concerning the quantitative difference of the protein level of the proteins detected in the sample subjected to investigation with respect to the reference works and the anamnestic and biographical data of the subject diagnosed.

Advantageously, the predictive model integrated in the program according to step (c') is based on an algorithm capable of integrating the data related to the quantitative difference in the level of proteins detected in the samples subjected to investigation and in the reference samples, with the anamnestic, biographical and gender (male or female) data of the subject subjected to investigation; the algorithm will compare the patient's data with a generally accepted baseline in healthy individuals.

The method of the invention was tested on a murine hepatic fibrosis model.

It was surprisingly observed that the aforementioned proteins have a significantly increased or decreased level in 2 different experimental sessions, each performed on multiple replicates, performed in hepatic fibrosis model mice in which surgical ligation of the bile duct was induced (BDL mice), with respect to the level of the same proteins in healthy mice (SHAM control mice). The first session relates to the analysis of the serum from mice at 8 days after surgical ligation of the bile duct (8BDL), thus at the earliest level of disease; the second session relates to the analysis of the serum from mice at 15 days after treatment (BDL mice), thus at overt disease.

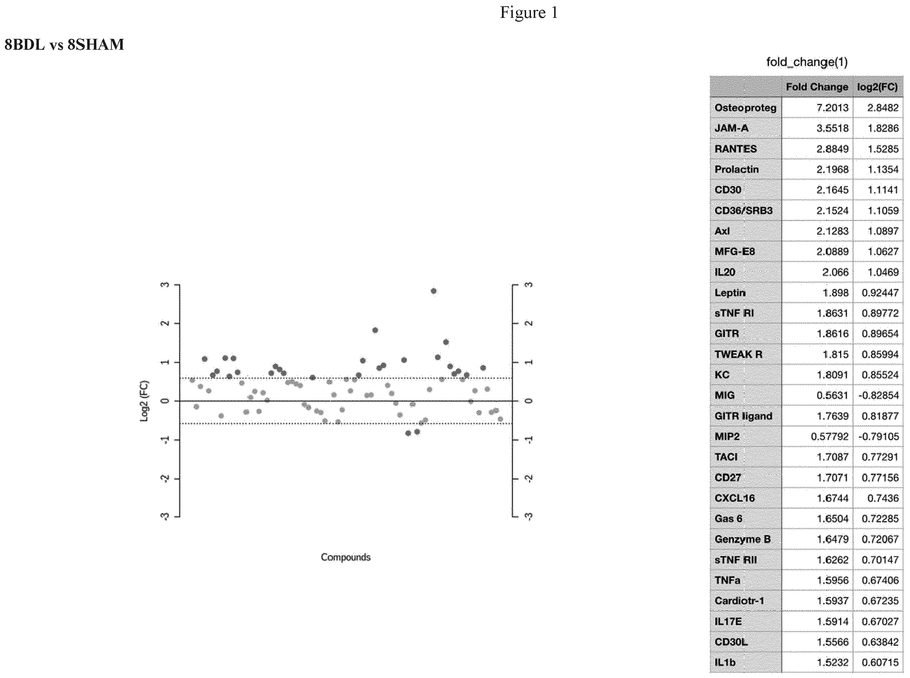

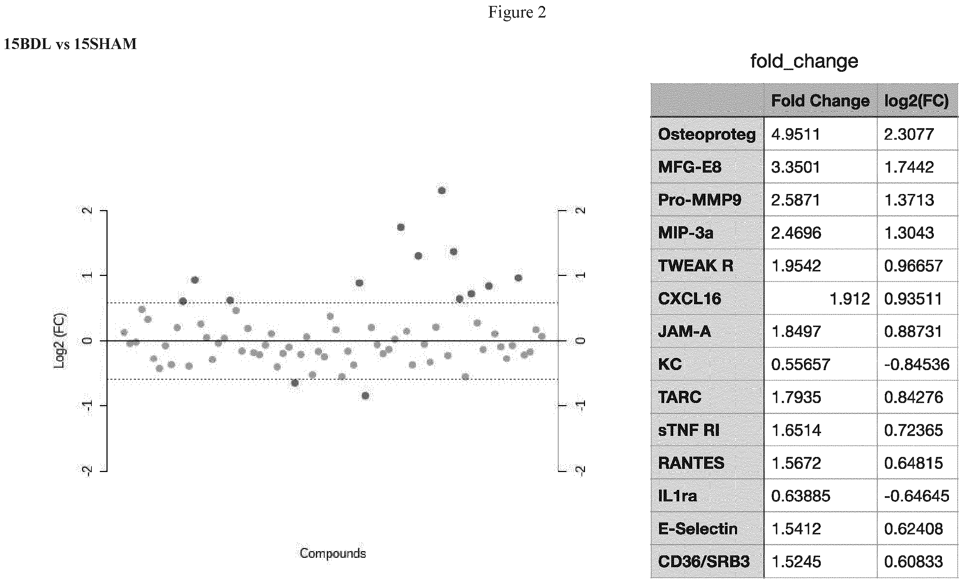

In particular, it was observed that of the 96 proteins analyzed, surprisingly some were found to be significantly altered (with a fold change > 1.5) in hepatic fibrosis model mice with respect to control mice. The *fold change* of the proteins according to the method for the 8BDL and 15BDL vs. 8 and 15SHAM mice is set forth in Figures 1 and 2. The data obtained from 8BDL vs. 8SHAM mice are the averages obtained from 3 mice in each group, whereas the data obtained from 15BDL vs. 15SHAM mice are the average of 4 animals per group.

The *"fold change"* is the measure that describes how much a measured quantity changes in two samples. It is defined as the ratio of the two quantities detected in two samples, which, in the present case, are the samples of BDL hepatic fibrosis model mice and control mice, not affected by SHAM hepatic fibrosis. A positive *fold change* > 1.5 is considered relevant for the subsequent statistical analyses, as is a negative *fold change* > 0.5.

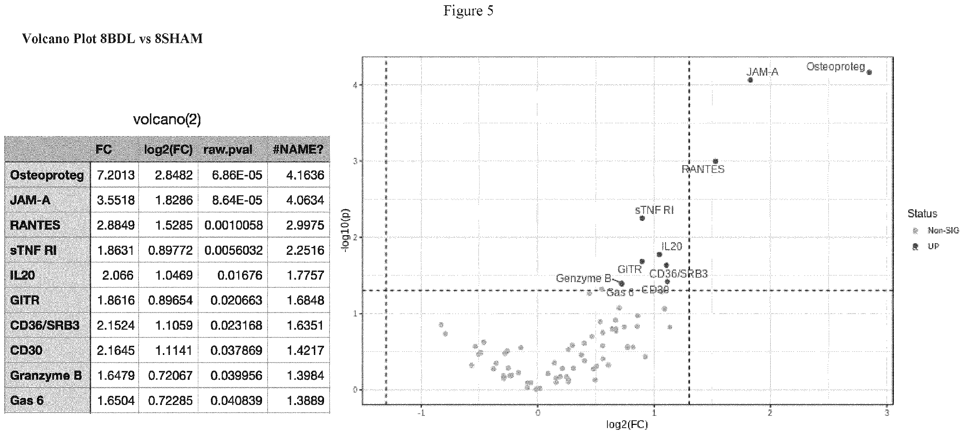

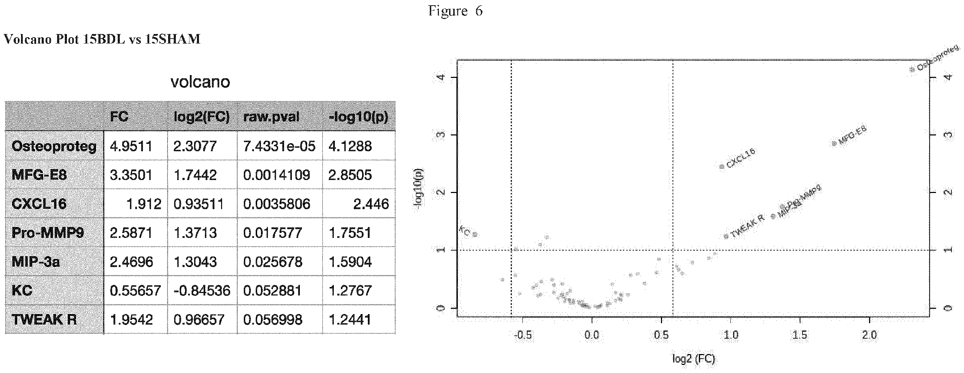

In Figures 5 + 6, the whole 16 proteins that were found to have a positive *fold change* >1.5 or a negative *fold change* > 0.5 are highlighted, combined with respect to the t-test analysis and by the Volcano plot statistical technique in the 3 experiments on 8BDL mice and the 4 experiments on 15BDL mice. The deviation from the control group can be positive, as for most of the proteins highlighted herein, or negative, as for KC (Figure 6), implying that this protein is under-regulated in the 15BDL mice. Of note, this protein was up-regulated at the early level (8BDL mice) although not in a statistically significant manner (Figure 1).

Figure 1 and 2 should be read by considering the number of times the protein level of the individual proteins is higher or lower in the hepatic fibrosis model mice with respect to healthy mice. By way of example, the quantitative level of Osteoprotegerin protein is 7.2-fold higher in 8BDL hepatic fibrosis model mice and 4.9-fold higher in 15BDL mice in comparison with the respective groups of healthy SHAM control mice. Furthermore, said protein was significant at statistical analysis of the Student-t in both groups (Fig. 3 and 4). Therefore, said protein proved to be a reliable and specific biomarker for the liver diseases affected by hepatic fibrosis, in particular for the cholestasis-induced hepatic fibrosis and also at an early stage, being the highest difference at 8 days in comparison with 15 days of disease.

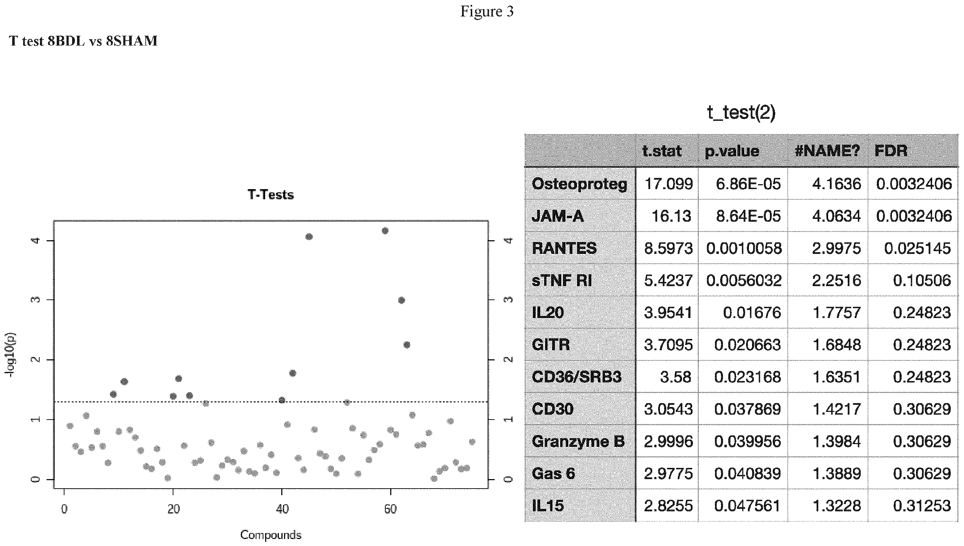

For example, the JAM-A protein is 3.5-fold higher in the experiment with 8BDL mice and 1.8-fold higher in the experiment with 15BDL mice with respect to the healthy controls. The same protein is highly significant at Student-t for the 8BDL mice (Fig. 3) and is also highlighted as being of strong interest at the volcano plot analysis for the 8BDL mice (Fig. 5), thus of interest as an early marker.

Therefore, the determination of the level of, for example, the osteoprotegerin protein as well as JAM-A and other proteins that have shown a significant *fold change* combined with a significant statistical evaluation, in isolated samples from diseased subjects, with respect to samples from healthy subjects, is certainly advantageous for the purposes of diagnosis.

In a preferred embodiment, step (a) or (a') of the method comprises determining, within one or more samples isolated from a subject, the protein level of Osteoprotegerin, JAM-A, RANTES, sTNF RI and CD36/SRB3. In fact, these molecules were found to have a significant *fold change* in both experimental sessions, with treatment of 8 and 15 days, together with a strong significance to the *Student-t* and *Volcano Plot* analysis performed on the samples from the treatment at 8 days (thus earlier).

Thus, the method according to the invention enables detecting the presence of hepatic fibrosis even when it is still at an early state, thanks to detecting the level of a pool of proteins that have been shown to be specific biomarkers of hepatic fibrosis. Therefore, said method allows an early and especially non-invasive diagnosis of the hepatic fibrosis.

Therefore, said method allows an early and especially non-invasive diagnosis of the hepatic fibrosis.

Further subject-matter of the present invention is a kit for the *in vitro* diagnosis of hepatic fibrosis, comprising means to quantitatively determine the levels of at least 3, preferably at least 5 proteins, selected from Osteoprotegerin, JAM-A and RANTES, sTNF RI, IL20, GITR, CD36/SRB3, CD30, Granzyme B, Gas 6, MFG-E8, CXCL16, Pro-MMP9, MIP-3a and TWEAK R.

These are the proteins that were found to be most over-regulated in the tests carried out (see Experimental Section), i.e. with a greater difference between the samples from mice affected with fibrosis and healthy mice.

According to another embodiment, the kit of the invention also comprises means to quantitatively determine the levels of KC, a protein that, as mentioned, has been found to be statistically significantly down-regulated in the tests carried out in the mice at 15 days (15BDL).

According to a preferred embodiment, the kit of the invention comprises means for performing an ELISA assay for the quantitative determination of the proteins as defined herein.

According to a preferred embodiment, the kit of the invention comprises means for performing a protein array assay, for example a protein microarray for the quantitative determination of the proteins as defined herein.

According to a preferred embodiment, the kit of the invention comprises primary antibodies specific to the Osteoprotegerin, JAM-A, RANTES, sTNF RI, IL20, GITR, CD36/SRB3, CD30, Granzyme B, Gas 6, MFG-E8, CXCL16, Pro-MMP9, MIP-3a, TWEAK R and KC proteins, and secondary antibodies directed against said proteins or against said primary antibodies, said secondary antibodies being marked with an enzyme biomarker, at least one buffer solution, and other suitable means. Alternatively, it is possible to prepare a multiplex ELISA assay that identifies all the different proteins that you want to evaluate in one tube only, thus reducing the time of execution.

Preferably, the kit of the invention comprises means adapted for use in an ELISA assay for the purpose of determining the level of one or more of the aforementioned proteins, preferably 3 or more, more preferably at least 5 of said proteins. According to an embodiment, said at least 5 proteins are selected from Osteoprotegerin, JAM-A, RANTES, sTNF RI, IL20, GITR, CD36/SRB3, CD30, Granzyme B and Gas 6, even more preferably at least one selected from Osteoprotegerin, JAM-A, RANTES, sTNF RI and CD36/SRB3. The kit of the invention may comprise means for performing an ELISA assay in each of the variants of said assay, i.e. direct ELISA, indirect ELISA, sandwich ELISA and competitive ELISA.

According to a preferred embodiment, the kit of the invention comprises means adapted for use in an ELISA assay in order to determine the level of Osteoprotegerin, JAM-A and RANTES proteins, and possibly KC protein.

According to another of its aspects, subject-matter the invention is the use of a kit according to any one of the embodiments described above, for the *in vitro* diagnosis of the hepatic fibrosis, preferably cholestasis-induced hepatic fibrosis.

According to another of its aspects, subject-matter of the invention is the use of a kit according to any one of the above-described embodiments, for implementing the method of the invention.

The kit of the invention may further comprise a program based on an algorithm allowing to integrate data relating to the protein level detected in one or more samples isolated from the subject diagnosed with anamnestic data and biographical data (sex and age) of said subject, so to process them in order to make a diagnosis of hepatic fibrosis.

According to another of its aspects, subject-matter of the invention is a panel of proteins comprised of at least 3, preferably at least 5 proteins selected from Osteoprotegerin, JAM-A e RANTES, sTNF RI, IL20, GITR, CD36/SRB3, CD30, Granzyme B, Gas 6, MFG-E8, CXCL16, Pro-MMP9, MIP-3, TWEAK R and KC, for its use as a biomarker of hepatic fibrosis, preferably cholestasis-induced hepatic fibrosis.

According to a preferred embodiment, said panel consists of Osteoprotegerin, JAM-A, RANTES, sTNF RI, IL20, GITR, CD36/SRB3, CD30, Granzyme B, Gas 6 and KC, even more preferably at least one selected from Osteoprotegerin, JAM-A, RANTES, sTNF RI and CD36/SRB3.

The use of the protein panels as defined above in the kit of the invention constitutes a further subject-matter of the invention.

### DESCRIPTION OF THE FIGURES

Figure 1 shows the univariate analysis of the *fold change* data obtained with "mouse cytokines antibody array" from the mean of 3 mice with ductus ligatus (8BDL) after 8 days of treatment versus 3 SHAM control mice (8 SHAM). Only the protein data found to be relevant, i.e. with a positive fold change> 1.5 and negative fold change <0.5, are selected.
Figure 2 shows the univariate analysis of the *fold change* data obtained with "mouse cytokines antibody array" from the mean of 4 mice with ductus ligatus (15BDL) after 15 days of treatment versus 4 SHAM control mice (15SHAM). Only the protein data found to be relevant, i.e., with a positive fold change> 1.5 and negative fold change <0.5, are selected.
Figure 3 shows the results of the *Student-t* analysis applied to the protein change data obtained with "mouse cytokines antibody array" from the mean of 3 mice with ductus ligatus (8BDL) after 8 days of treatment versus 3 SHAM control mice (8SHAM). Only the protein data found to be statistically significant, i.e., p < 0.05, are selected. However, if we correct for the false discovery rate (FDR) parameter < 0.05, only the first 3 (Osteoprotegerin, JAM-A and RANTES) are relevant.

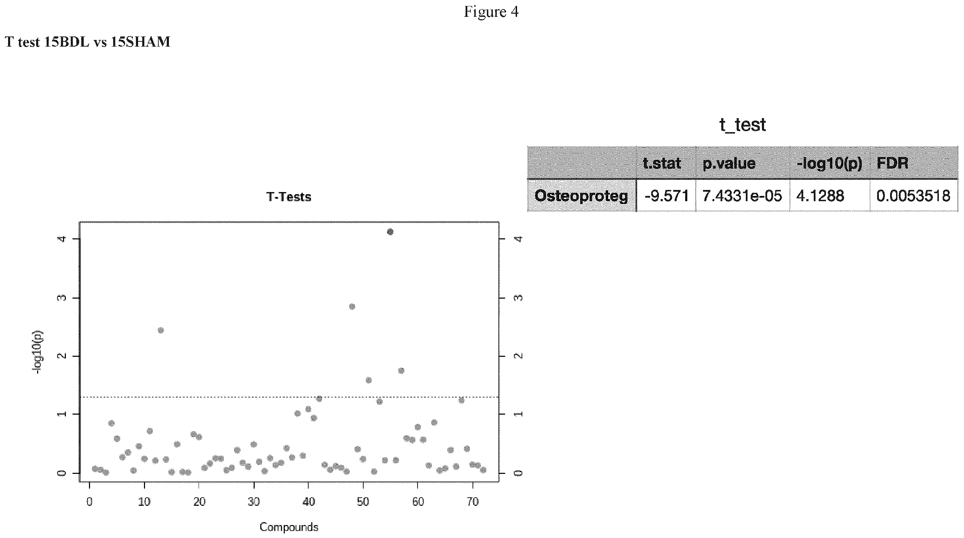

Figure 4 shows the results of the *Student-t* analysis applied to the protein change data obtained with "mouse cytokines antibody array" from the mean of 4 mice with ductus ligatus (15BDL) after 15 days of treatment versus 4 SHAM control mice (15SHAM).
Only the protein data found to be statistically significant, i.e., p < 0.05, which in the specific case turns out to be only one, osteoprotegerin, are selected.
Figure 5 shows the results of the *Volcano plot* statistical analysis combining the fold changes selected in Figure 1 with the *Student-t* in Figure 3. 10 proteins are identified that significantly increase in the mice at early levels, after 8 days of disease induction, with respect to the untreated controls.
Figure 6 shows the results of the *Volcano plot* statistical analysis combining the fold changes selected in Figure 2 with the *Student-t* in Figure 4. 6 proteins are identified that significantly increase in the mice after 15 days of disease induction with respect to the untreated controls, and one protein (KC) that decreases. Out of these 7 proteins, only 1 (osteoprotegerin) overlaps with the previous group identified in Figure 5.

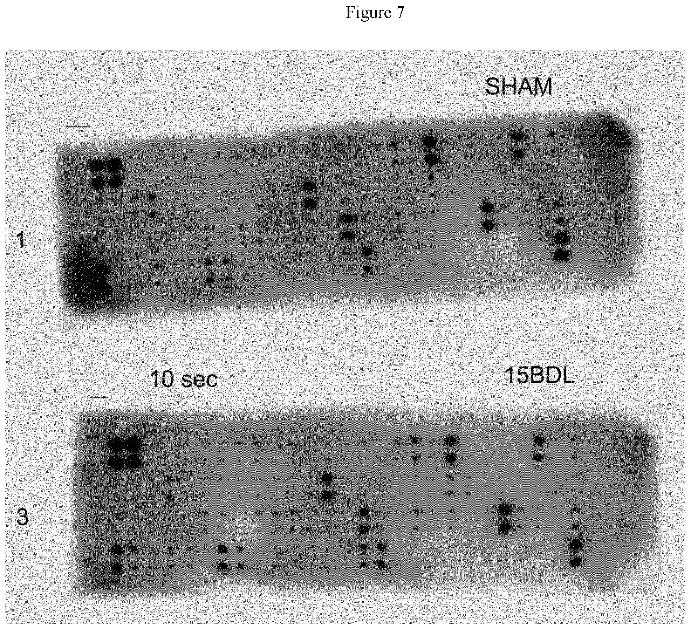

Figure 7 shows an example of a membrane from the Abcam Cytokine Array - Mouse Cytokine Antibody Array (Membrane, 96 Targets) kit (ab193659), treated with the serum from a single mouse 15 BDL vs. a SHAM mouse.

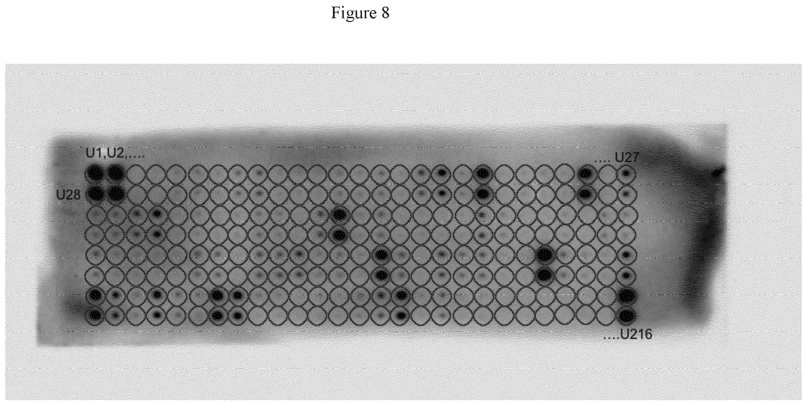

Figure 8 shows an example of how the ImageLab^{™} v6.00 software (BioRad) acquires and processes the membrane image through a mask to transform the pixels into a given amount of *fold change.*

### EXPERIMENTAL SECTION

### Example 1

### Animal model for the cholestasis-induced hepatic fibrosis

In order to identify specific biomarkers for the diagnosis of hepatic fibrosis, animal models mimicking human cholestasis-induced hepatic fibrosis were used.

In particular, C57BL/6 strain mice were used, in which surgical ligation of bile duct (BDL) was induced at different times (until the first signs of hepatic fibrosis appeared). Bile duct ligation was induced for 8 days (group 8 BDL) and for 15 days (group 15 BDL). The protocol followed for the bile duct ligation is that of Tag and colleagues (Tag CG et al., Bile duct ligation in mice: induction of inflammation liver injury and fibrosis by obstructive cholestasis. J Vis Exp. 2015; e52438). After the injection of the analgesic, surgical opening of the mouse on the abdomen was performed and the liver was lifted with a hydrated cotton swab so that the ileum was clearly visible. The bile duct was exposed via caudal moment of the intestine and was separated from the portal vein and hepatic artery by using micro-serrated forceps. A 5-0 suture was placed around the bile duct and secured with two surgical knots. A second ligation higher up was applied in the same way without dissecting the bile duct between the ligations. The first abdominal layer was closed with 6.0 silk suture thread, and the second abdominal layer was closed with Michel clip 7.5X1.75.

### Example 2

### Biomarker determination of hepatic fibrosis by protein array

The serum from 4 groups of mice, each comprised of 4 mice for the experiment carried out 14 days after bile duct ligation (4 15BDL mice vs. 4 15SHAM mice) and 3 mice for the experiment carried out 8 days after bile duct ligation (3 8BDL mice vs. 3 8SHAM mice), was analyzed for detecting proteins having a different quantitative level in hepatic fibrosis model mice and control mice. The 3 groups of mice used are: the 15BDL and 8BDL mice obtained according to the procedure stated in Example 1 and control 15 and 8 SHAM mice, i.e. mice of the C57BL/6 strain not suffering from hepatic fibrosis. In particular, an array was used to assess the level of 96 soluble proteins, said array being the Abcam Cytokine Array - Mouse Cytokine Antibody Array (Membrane, 96 Targets) (ab193659) https://www.abcam.com/cytokine-array-mouse-cytokine-antibody-array-membrane-96-targets-ab193659.html. The Abcam array used features primary antibodies specific for a panel of 96 soluble proteins of the cytokine family or other soluble protein molecular markers adsorbed on a membrane, biotin-conjugated secondary antibodies also specific for a panel of 96 proteins and peroxidase-labeled streptavidin. Some of the proteins tested by Abcam protein array and the results for them are shown in Figure 1 and 2. The serum samples isolated from the 4 groups of mice were treated according to the protocol for using said protein array and reacted with the membrane of the Abcam array. The serum samples were reacted with the membrane overnight after which incubation of said membrane with biotin-conjugated secondary antibodies was carried out again for 2 hours. After 2 hours, the membrane is incubated with labeled streptavidin, and the labeled streptavidin reacts with biotin conjugated to secondary antibodies that have bound to the cytokine, if present in the serum of the mice.

### Experimental conditions performed:

2 membranes were saturated with Blocking buffer. 210 µl per sample were used to have comparable conditions with the previous experiments (dilution 1/9.5).

The protocol was performed according to the manufacturer's instructions, opting for the incubation of O.N. sera at 4°C and introduction of an additional extensive wash.

The subsequent incubations (biotin-mouse anti-cytokine and HRP-streptavidin) were performed with 2-hour times at room temperature. After the treatment, the membrane will show more or less intense spots of color where the antibody has bound to the protein present in the serum. An example of what the membranes look like is shown in Figure 7.

After ON incubation, the sera diluted in blocking buffer were recovered and stored at -20°C.

After 2 hours from the incubation of the membrane with the labeled streptavidin, the membrane reading was performed. Images were analyzed with ImageLab^{™} v6.00 software (BioRad) by using the spot analysis mask generated in the analysis of the first PB-01/2020 experiment, in order to have comparable area values. Image acquisition was performed with ChemiDoc^{™} Touch Imaging System instrument. The arrays were acquired in pairs to have the same magnification rate as the previous experiments. The acquisition was done with auto exposure, which was found to be 1.16 sec; subsequently acquisitions were made with manual exposures of 1, 2, 4, 8, 10, 12 and 20 seconds.

The results of the protein array repeated in multiple sessions show the presence of a panel of over-regulated proteins, with a 15BDL/SHAM ratio significantly higher than 1 or lower than 0.5. The readout of said membrane showed that the level of 16 proteins out of the 96 studied appeared to be significantly modulated in the sera of hepatic fibrosis model mice with respect to the control mice, in particular after statistical analysis of the Volcano Plot type. Out of said 16 proteins being Osteoprotegerin, JAM-A, RANTES, sTNF RI, IL20, GITR, CD36/SRB3, CD30, Granzyme B, Gas 6, MFG-E8, CXCL16, Pro-MMP9, MIP-3a and TWEAK R and KC, 15 are clearly over-regulated, whereas only one, KC, is under-regulated in mice with the disease at 15 days and over-regulated (although not significantly) in mice with early-stage disease at 8 days. In particular, it has been observed that 5 out of these 16 proteins have a higher quantitative difference and with greater statistical significance in the 8BDL mice, then at the earliest stage of the disease, while they are still over-regulated in the mice at 15 days, said 5 proteins being osteoprotegerin, JAM-A RANTES, sTNF RI, CD36/SRB3I. Osteoprotegerin was statistically significant in both groups of mice, at 8 and 15 days of disease.

## Claims

1. A method for making an *in vitro* diagnosis of hepatic fibrosis, comprising:
a. measuring the levels of at least 3 proteins, preferably at least 5 proteins, selected from
Osteoprotegerin, JAM-A, RANTES, sTNF RI, IL20 GITR, CD36/SRB3, CD30, Granzyme B, Gas 6, MFG-E8, CXCL16, Pro-MMP9, MIP-3 and TWEAK R, in one or more isolated biological samples of a subject;
b. comparing said levels with one or more reference values, said reference values being the levels of said proteins in a healthy subject and/or in a subject suffering from hepatic fibrosis;
wherein
- the over-regulation of said at least 3, preferably at least 5 proteins, with respect to the levels of said proteins in a healthy subject, and/or
- the substantial equivalence of the levels of said at least 4, preferably said at least 5 proteins with respect to the levels of said proteins in a subject suffering from hepatic fibrosis,
are indicative of hepatic fibrosis and/or predisposition or progression of the condition of hepatic fibrosis.

2. The method according to claim 1, **characterized in that** said at least 3 proteins, preferably at least 5 proteins, comprise Osteoprotegerin, JAM-A, RANTES, sTNF RI, IL20 GITR, CD36/SRB3, CD30, Granzyme B and Gas 6.

3. The method according to claim 1 or 2, **characterized in that** said at least 3 proteins, preferably at least 5 proteins, are Osteoprotegerin, JAM-A, RANTES, sTNF RI, and CD36/SRB3.

4. The method according to any one of the preceding claims, **characterized in that** it also comprises measuring the KC protein, wherein
- the down-regulation of said protein with respect to the levels of said protein in a healthy subject, and/or
- the substantial equivalence of the levels of said protein with respect to the levels of said protein in a subject suffering from hepatic fibrosis,
are indicative of hepatic fibrosis and/or predisposition or progression of the condition of hepatic fibrosis.

5. The method according to any one of the preceding claims, further comprising a step (c) of processing the obtained comparison data of step (b) by a program comprising a predictive model.

6. The method according to the preceding claim, wherein said program integrates information related to personal and anamnestic data of the subject to be subjected to said diagnosis with the comparison data obtained in step (b) of the method.

7. The method according to any one of the preceding claims, **characterized in that** step (a) is carried out by ELISA assay.

8. The method according to any one of claims 1 to 6, **characterized in that** step (a) is carried out on an analysis platform of the protein level, preferably a platform of protein array.

9. A kit for the *in vitro* diagnosis of hepatic fibrosis, comprising means to quantitatively determine at least 3 proteins, preferably at least 5 proteins, selected from Osteoprotegerin, JAM-A, RANTES, sTNF RI, IL20, GITR, CD36/SRB3, CD30, Granzyme B, Gas 6, MFG-E8, CXCL16, Pro-MMP9, MIP-3 and TWEAK R.

10. The kit according to claim 9, **characterized in that** it comprises means for performing an ELISA assay.

11. The kit according to claim 9 or 10, **characterized in that** it comprises means for performing a "protein array".

12. The kit according to claim 9 or 10, **characterized in that** it comprises means for performing a multiplex ELISA assay and it comprises specific antibodies to at least 3 proteins, preferably at least 5 proteins, selected from Osteoprotegerin, JAM-A, RANTES, sTNF RI, IL20, GITR, CD36/SRB3, CD30, Granzyme B, Gas 6, MFG-E8, CXCL16, Pro-MMP9, MIP-3 and TWEAK R.

13. The kit according to any one of claims 9 to 12, **characterized in that** it comprises means and/or specific antibodies also to quantitatively determine the KC protein.

14. Use of the kit according to any one of claims 9 to 13, for the *in vitro* diagnosis of the hepatic fibrosis, preferably cholestasis-induced hepatic fibrosis.

15. A panel of proteins comprised of at least 3 proteins, preferably at least 5 proteins, selected from Osteoprotegerin, JAM-A, RANTES, sTNF RI, IL20, GITR, CD36/SRB3, CD30, Granzyme B, Gas 6, MFG-E8, CXCL16, Pro-MMP9, MIP-3, TWEAK R and KC, for its use as a biomarker of hepatic fibrosis, preferably cholestasis-induced hepatic fibrosis.
